# EUROPEAN PATENT APPLICATION

(11) **EP 1 800 751 A1**
(43) Date of publication of application: **27.06.2007**
(21) Application number: 05026062.9
(22) Date of filing: 30.11.2005
(51) Int. Cl.: B01L 3/00, A61B 5/151, G01N 33/50

(54) **Lateral-flow test device for liquid samples**

(71) Applicant: The Jordanian Pharmaceutical Manufacturing Co., 11710 Naor (JO)
(72) Inventor: Murshed, AbdelQuader Mohammed, 11118 Amman (JO); Dr. Badwan, Adnan, 11185 Amman (JO)
(74) Representative: Winkler, Andreas Fritz Ernst

(57) **Abstract**

The present invention refers to a lateral-flow test device comprising means for releasing a test buffer harboured by the test device such that a sample to be tested, preferably body fluid, is exposed to the test buffer. The invention also refers to a whole blood lateral-flow test device additionally comprising a lancet and means for ejecting the lancet. Preferably, the analyte in the sample to be tested, e.g. whole blood, is an antibody or antigen. Furthermore, the invention refers to uses of said test devices.

## Description

The present invention refers to a lateral-flow test device comprising means for releasing a test buffer harboured by the test device such that a sample to be tested, preferably body fluid, is exposed to the test buffer. The invention also refers to a whole blood lateral-flow test device additionally comprising a lancet and means for ejecting the lancet. Furthermore, the invention refers to uses of said test devices.

### Background of the invention

In recent years the *in vitro* diagnostics (IVD) industry has made enormous efforts to develop membrane-based lateral-flow tests. Such tests have found applications in both clinical and non-clinical fields (1). A clinical utility of this test format has been shown for more than 150 different analytes, and many of them are target now of commercially available diagnostic products (3). The wide range of applications for such devices has been reviewed (1, 2).

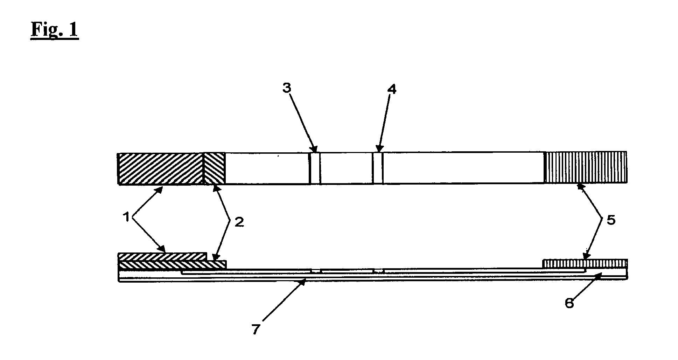

Membrane-based lateral-flow test devices, e.g. rapid-flow immunochromatographic test devices, are made up of a number of components commonly including a sample pad, a conjugate pad, a membrane that incorporates capture reagents, and an absorbent pad (see Figure 1). In practice, the user dispenses a patient sample (usually urine, plasma/serum or whole blood) onto the sample pad. The sample then flows through the sample pad into the conjugate pad, where it mixes with and releases the conjugate reagent. This mixture then flows across the membrane, where it binds with the test and control reagents. When the mixture binds to the reagent that forms the test line, a positive result is indicated. The colour intensity of the test line is mostly proportional to the concentration of analyte in the sample. Excess sample that flows beyond the test and control lines is taken up in the absorbent pad.

Membrane-based lateral-flow test devices for diagnostic purposes are easy to operate and thus do not only contribute to the comfort of professional users, e.g. medical stuff, but also allow the operation by non-professionals users, e.g. most patients.

However, one serious problem associated with the use of lateral-flow test devices when serum, plasma or whole blood is used as the sample to be tested is the application of the test buffer solution. In consumer test devices currently available, the buffer is generally provided separately in a vial or dropper. This component is preferred to be outside of the device in order to avoid any possible damage of the pouch, strip or desiccant. There is also the risk of releasing some buffer drops from the dropper. Moreover, a very serious problem is the risk of inaccurate measuring out the required buffer drops to be applied to the test device. Using extra or less than the required buffer quantity, however, could affect the results accuracy.

Another problem is that a separate lancet must be used in the case that whole blood should be delivered from the finger tip. The protective shield covering the lancet could be damaged or removed during storage. Furthermore, when a test device as currently available is taken with the user, lancets have to be taken additionally. This is less convenient since the user has to think of more than one thing, and in case that the lancets are left behind, a necessary test may be even impossible.

In all currently available rapid-flow immunochromatographic test devices in the IVD market around the globe, the user is provided with instructions, e.g. by the package insert, such as: "Use the provided lancet " or "Use the provided buffer". Thus, the final consumer is induced to use a test device and a buffer containing vessel and, if necessary, lancets. The need of additional parts, however, may be more or less uncomfortable, and in certain cases a correct use of the test device is even threatened.

Consequently, there is still a need in the art to provide a lateral-flow test device allowing a more convenient operation and thus, enabling more reliable test results.

Therefore, it was an object of the present invention to provide a lateral-flow test device having such improved properties.

### Summary of the invention

The object of the present invention is solved by a lateral-flow test device comprising means for exposing a sample to be tested for at lest one analyte to a test buffer, characterised in that said test buffer is harboured in the test device.

In one embodiment, the means for exposing the sample to the buffer comprise a buffer release button (12).

In one embodiment, the sample is a body fluid, selected from the group comprising serum, plasma and whole blood.

In a preferred embodiment, the lateral-flow test device further comprises a lancet (13), a lancet protective shield (15), and means for ejecting said lancet.

In a particularly preferred embodiment, the means for ejecting the lancet comprise a lancet ejection button (14).

In a most preferred embodiment, the lateral-flow test device comprises a lancet, a lancet protective shield and means for ejecting said lancet, preferably a lancet ejection button, and the sample is whole blood.

In one embodiment, the analyte is an antibody or antigen in a sample, preferably whole blood.

The object of the present invention is solved by a use of a lateral-flow test device for detecting and/or semi-quantifying at least one analyte in a sample.

In one embodiment, the sample is a body fluid, selected from the group comprising serum, plasma and whole blood.

The object of the present invention is further solved by a use of a lateral-flow test device for detecting and/or semi-quantifying at least one analyte in whole blood.

The object of the present invention is further solved by a use of a lateral-flow test device for monitoring the blood analyte level.

In one embodiment of the above uses, the analyte is an antibody or antigen in a sample, preferably whole blood.

In one embodiment, the presence of the antibody or antigen in a sample, preferably whole blood, is associated with a disease, preferably a bacterial, viral or parasitic infection.

In further embodiments, all analytes detectable in whole blood, plasma, serum and other body fluids are considered, such as tumour markers, e.g. the prostate-specific antigen (PSA), steroidal and non-steroidal hormones, ethanol and its metabolites, etc. Also considered are human chorionic gonadotropin (hCG) antigen, *Helcicobacter pylori* antibodies, HIV antibodies, human lutropin (hLH), human follitropin (hFSH), C-reactive protein, thyroid stimulating hormone (TSH), influenza A virus, influenza B virus, adenovirus, rotavirus, creatine kinase MB isoenzyme (CK-MB), myoglobin, *Chlamydia,* malaria pathogen, alpha-fetoprotein (AFP), carcinoembryonic antigen (CEA), tuberculosis (TB) antigens or antibodies, *Brucella,* troponin C, troponin I, troponin T, *Toxoplasma,* rubella virus, *Salmonella,* Pan *Salmonella, Listeria,* marihuana, methamphetamine, morphine, amphetamine, cocaine, phencyclidine, barbital, methadone, oxycodone, ethadone, etc.

### Detailed description of the invention

Embodiments of the present invention shall be now exemplified in more detail referring to the attached figures and the following examples.
*Figure 1* shows a top and side view of a typical rapid-flow immunochromatographic test device comprising a sample pad (1), a conjugate pad (2), a capture (test) zone (3), e.g. a membrane that incorporates at least one capture reagent, a control (negative) zone (4), an absorbent pad (5), an adhesive (6), and a plastic backing (7).
*Figure 2* shows one embodiment of the lateral-flow test device of the present invention comprising a humidity indication hole (8), which indicates whether the test device has been spoiled by moisture and/or serves for indication of the appropriate reading time, a control indication hole (9), a result indication hole. (10), a sample hole (11), and a buffer release button (12).
*Figure 3* shows one embodiment of the lateral-flow test device of the present invention further comprising a lancet (13), a lancet ejection button (14), and a protective shield (15).
*Figure 4* demonstrates step-by-step the use and operation of one embodiment of the lateral-flow test device of the present invention using whole blood as a sample to be tested.

### EXAMPLES

### EXAMPLE 1: Serum or plasma test device

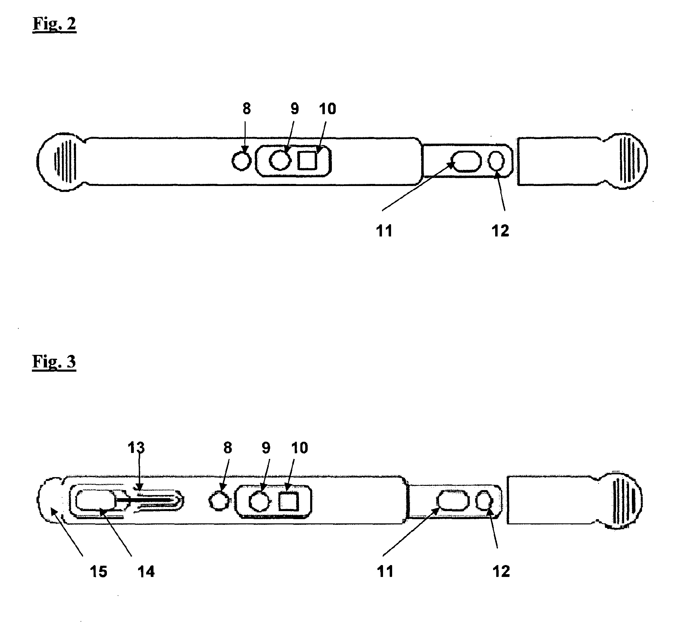

In one embodiment, a lateral-flow test device contains the test buffer, e.g. in a buffer storage cavity formed by the device or in a special bag located in the interior of the device. The buffer can be released by pressing a buffer release button (12), whereupon the buffer is contacted with a sample applied previously to the sample hole (11) (see *Fig. 2-4*).

This type of test device is preferably used for testing serum or plasma. However, this test device configuration can be used as well with whole blood obtained, for example, by using an external lancet or by vein puncture.

### EXAMPLE 2: Whole blood test device

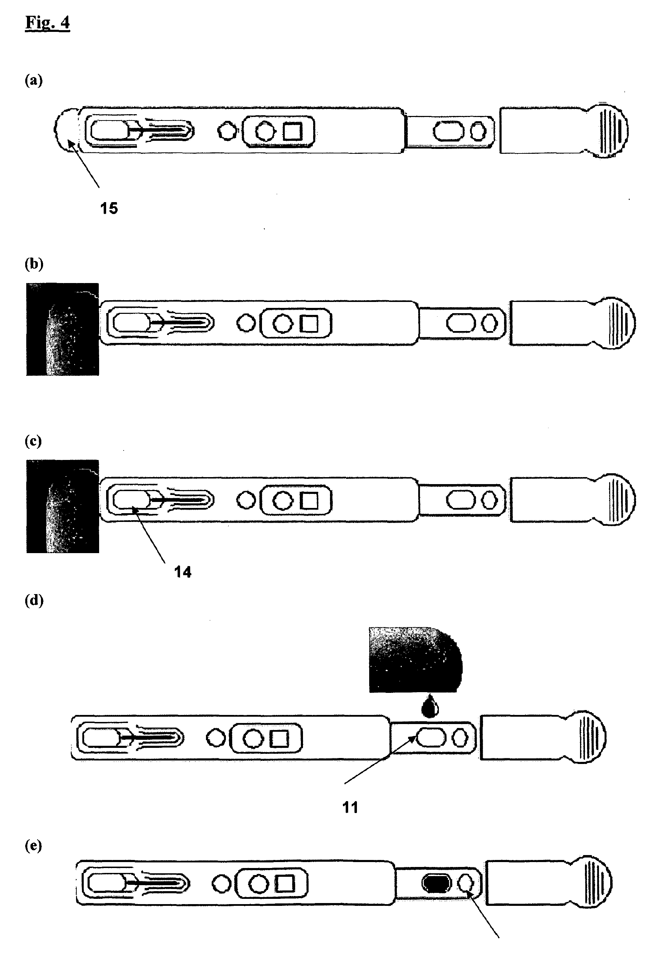

In one embodiment, the lateral-flow test device described in Example 1 above further provides a lancet (13) which can be ejected by pressing a lancet release button (14) inducing a lancet self-release mechanism (see *Fig. 3, 4*). Prior to use, e.g. during storage of the test device, the site where the lancet is ejected or the area more or less surrounding said site, i.e. a region which comes into contact with the finger tip, is covered by a protective shield (15).

This type of test device is preferably used for testing whole blood freshly obtained from the finger tip by using the lancet being part of the test device.

### EXAMPLE 3: Use and operation of a whole blood test device

When using the whole blood test device described in Example 2, the following steps are to be carried out:
(a) Turn the protective shield chlockwise to push the lancet's needle into the body of the device.
(b) Remove the protective shield, e.g. by turning.
(c) Put your well-cleaned finger at the end of the device where the protective shield has been removed.
(d) Press on the ejection button for lancet ejection.
(e) Apply blood drops to the sample hole.
(f) Press the buffer release button.
(g) Wait for colour change of the reading-time indicating hole.
(h) Read Results.
(i) Collect the device, lancet protective shield and the device cap in the aluminium foil for discard.

After contacting the sample and buffer, the analyte detection reaction is initiated and run. Then, after a pre-determined reactions time, the test result is ready for read.

### References:

(1) J Chandler, N Robinson, and K Whiting, "Handling False Signals in Gold-Based Rapid Tests", IVD Technology 7, no. 2 (2001): 34-45; http://www.devicelink.com/ivdt/archive/0 I /03/002.html.
(2) J Chandler, T Gurmin, and N Robinson, "The Place of Gold in Rapid Tests", IVD Technology 6, no. 2 (2000): 37-49; http://www.devicelink.com/ivdt/archive/00/03/004.html
(3) TC Tisone et al., "Image Analysis for Rapid-Flow Diagnostics", IVD Technology 5, no. 5 (1999): 52-58; http://www.devicelink.com/ivdt/archive/99/09/010.html

## Claims

1. A lateral-flow test device comprising means for exposing a sample to be tested for at lest one analyte to a test buffer, **characterised in that** said test buffer is harboured in the test device.

2. The lateral-flow test device according to claim 1, wherein the means for exposing the sample to the buffer comprise a buffer release button (12).

3. The lateral-flow test device according to claims 1 or 2, wherein the sample is a body fluid, selected from the group comprising serum, plasma and whole blood.

4. The lateral-flow test device according to any of the preceding claims, further comprising a lancet (13), a lancet protective shield (15), and means for ejecting said lancet.

5. The lateral-flow test device according to claim 4, wherein the means for ejecting the lancet comprise a lancet ejection button (14).

6. The lateral-flow test device according to claims 4 or 5, wherein the sample is whole blood.

7. The lateral-flow test device according to any of the preceding claims, wherein the analyte is an antibody or antigen.

8. A use of a lateral-flow test device according to claims 1 or 2 for detecting and/or semi-quantifying at least one analyte in a sample.

9. The use according to claim 8, wherein the sample is a body fluid, selected from the group comprising serum, plasma and whole blood.

10. A use of a lateral-flow test device according to any of claims 1, 2, 4 and 5 for detecting and/or semi-quantifying at least one analyte in whole blood.

11. A use of the lateral-flow test device according to any of claims 1, 2, 4 and 5 for monitoring the blood analyte level.

12. The use according to any of claims 8, 10 and 11, wherein the analyte is an antibody or antigen.

13. The use according to claim 12, wherein the antibody or antigen is associated with a disease, preferably a bacterial, viral or parasitic infection.
